(19) Europäisches Patentamt
European Patent Office
Office européen des brevets

(11) Publication number: **0 128 506**
**B1**

(12) # EUROPEAN PATENT SPECIFICATION

(45) Date of publication of patent specification: **07.10.87**

(51) Int. Cl.⁴: **C 07 C 19/08,** C 07 C 17/38

(21) Application number: **84106409.0**

(22) Date of filing: **06.06.84**

(54) **Removal of carbon monoxide from perfluoroalkanes.**

(30) Priority: **06.06.83 US 501108**

(43) Date of publication of application:
**19.12.84 Bulletin 84/51**

(45) Publication of the grant of the patent:
**07.10.87 Bulletin 87/41**

(84) Designated Contracting States:
**BE DE FR GB IT LU NL**

(56) References cited:

**CHEMICAL ABSTRACTS, vol. 82, no. 12, 24th March 1975, page 242, no. 76738k, Columbus, Ohio, USA**

(73) Proprietor: **E.I. DU PONT DE NEMOURS AND COMPANY**
**1007 Market Street**
**Wilmington Delaware 19898 (US)**

(72) Inventor: **Gumprecht, William Henry**
**2606 Stephenson Drive**
**Wilmington Delaware 19808 (US)**

(74) Representative: **Abitz, Walter, Dr.-Ing. et al**
**Abitz, Morf, Gritschneder, Freiherr von Wittgenstein Postfach 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 128 506 B1

**Description**

*Field of Invention*

This invention relates to a process for removing carbon monoxide from perfluoroalkanes.

*Background of the Invention*

Vapor-phase processes for the production of perfluoroalkanes, such as tetrafluoromethane ($CF_4$) and hexafluoroethane ($C_2F_6$), by fluorination of chlorocarbons with hydrogen fluoride commonly use chromium (III) oxide to catalyze the fluorination reaction. A side reaction in such processes is the formation of small amounts of carbon monoxide particularly as the chromium oxide loses catalytic activity. It is postulated that carbon monoxide production is either due to incomplete oxidation of carbonaceous solids resulting from thermal decomposition of the organic raw materials or to thermal dissociation of carbonyl halides, such as phosgene, that may be formed by interaction between catalyst and organic raw materials. While the levels of carbon monoxide are generally low (less than 2000 ppm), its separation from the perfluoroalkanes by distillation is difficult and requires large amounts of refrigeration.

It is known from Chemical Abstracts *82* (1975) 242, Abstract 76738k, to use manganese dioxide and cupric oxide as an oxidizing catalyst in a process for the removal of carbon monoxide.

Certain uses for perfluoroalkanes, such as plasma etching of semi-conductors, require a product of very high purity. The effectiveness of perfluoroalkanes in plasma etching is adversely affected by even trace amounts of carbon monoxide. It is, therefore, an object of this invention to provide an economical method for removing carbon monoxide from perfluoroalkanes. It is a further object to provide a continuous method for removing carbon monoxide that can be integrated into the overall process for the production of perfluoroalkanes. These and other objects will be apparent from the description of the invention provided herein.

*Summary of the Invention*

The present invention provides a process for the removal of carbon monoxide from perfluoroalkanes comprising contacting a gaseous mixture of the perfluoroalkane and carbon monoxide plus oxygen in a minor amount, sufficient to oxidize carbon monoxide in the perfluoroalkane to carbon dioxide, with a bed of granules consisting of 67 to 86% by weight of manganese dioxide and 33 to 14% by weight of cupric oxide to convert the carbon monoxide to carbon dioxide and separating the carbon dioxide that is formed from the perfluroalkane. Separation of the carbon dioxide is accomplished by passing the gas stream from the bed of $MnO_2/CuO$ particles through a bed of soda lime (a mixture of sodium hydroxide and calcium oxide) or sodium hydroxide deposited upon vermiculite which absorbs the $CO_2$ present in the gas mixture. It is also possible to remove $CO_2$ from the perfluoroalkane by passing the gas stream through a bed of molecular sieves. The preferred method of carbon dioxide removal is by contacting the perfluoroalkane/carbon dioxide mixture with a bed of sodium hydroxide deposited on vermiculite. It is also preferred to follow such contact with a drying step, such as contact with a bed of molecular sieves, to remove any moisture present in the gas steam. Molecular sieves having a pore size of 0.4 nm are suitable for drying as well as for carbon dioxide removal. Perfluoroalkanes that can be treated by the process of the present invention include tetrafluoromethane and hexafluoroethane. A particular advantage of the invention is that it provides a purification process that can be operated as part of a continuous process for the manufacture of perfluoroalkanes.

FIG. 1 of the attached drawing is a schematic diagram showing the use of the present invention in continuous plant processes for the purification of perfluoroalkane.

FIG. 2 of the attached drawing is a schematic diagram of laboratory equipment for removing carbon monoxide from perfluoroalkanes according to the present invention.

*Detailed Description of the Invention*

Referring to FIG. 1, line *1* carries a gas stream from the vapor-phase fluorination of chlorotrifluoromethane with HF in the presence of chromium oxide catalyst. The major component of this gas stream is tetrafluoromethane, but it will contain other ingredients including small amounts of unreacted starting materials and air that is present in the starting materials. Also, the presence of carbon monoxide is always a possibility, and since it can not be tolerated in certain applications, the steps to be described hereinafter are carried out. The stream in line *1* is passed through drying columns *2* and *3* which contain sodium aluminosilicate beads (0.4 nm (4A) molecular sieves) to remove traces of moisture that might be present. The pressure of the gaseous mixture exiting from column *3* is then increased to about 28.6 bar by compressor *4* before entering refrigerated fractionation column *5* where unreacted chlorotrifluoromethane in the mixture is separated and recycled back to the fluorinator (not shown) via line *6*. Undesirable low boiling inerts that may be present are separated from the tetrafluoromethane in condenser *7* and vented from the system via line *8*, and fluorinated methane product is returned to the fractionation column. The tetrafluoromethane fraction separated in column *5* which contains air and small amounts of CO is transferred via line *9* to column 10 where it passes through a mixture of manganese dioxide and cupric oxide particles which catalyzes the air oxidation of any carbon monoxide present to carbon dioxide. After contact with the particle bed in column *10*, the gaseous mixture passes via line *11* to column *12* which contains a particulate material capable of removing carbon dioxide from the gaseous tetrafluoromethane. A preferred material for this purpose is sodium hydroxide deposited on particles of vermiculite. Following carbon dioxide removal in column *12*, the tetrafluoromethane is carried via line *13* to column *14* where it is subject to contact with a drying agent, such as 0.4 nm (4A) molecular sieves. The purified tetrafluoromethane emerging from column *14* is then transferred via line *15* to storage facilities not shown.

The mixture of manganese dioxide and cupric oxide acts as a catalyst for the oxidation of carbon monoxide with oxygen in the air to carbon dioxide. Oxygen is present in adequate concentrations in perfluoroalkanes as they are produced to meet the stoichiometric requirements for the oxidation which proceeds according to the following equation:

$$2CO + O_2 \xrightarrow{MnO_2/CuO} 2CO_2$$

This reaction is exothermic and will occur quantitatively at ambient temperature and atmospheric pressure in the presence of the $MnO_2/CuO$ catalyst. Since the concentration of carbon monoxide in perfluoroalkanes is low, the temperature rise is only a few degrees Celsius. Operation at temperatures up to 200°C does not shorten the useful life of the catalyst nor adversely affect the perfluoroalkanes. It is contemplated that pressures above atmospheric (e.g., up to 18,2 bar) can be used in this reaction provided that materials of construction in the equipment are adequate.

In certain uses for perfluoroalkanes, such as plasma etching of semi-conductors, the presence of carbon dioxide is also undesirable making its removal essential. Since the initial oxidation of the carbon monoxide in the presence of the $MnO_2/CuO$ catalyst is performed under anhydrous conditions, conventional scrubbing techniques for carbon dioxide removal, such as aqueous caustic scrubbing, would add large amounts of water vapor which would have to be removed. Therefore, it is preferable to use techniques that can be carried out under essentially anhydrous conditions or conditions that do not introduce any substantial amount of water into the system. The removal of carbon dioxide by adsorption on molecular sieves, such as 0.4 nm (4A) molecular sieves, can be employed, but in cases where maximum carbon dioxide removal is desired, other techniques such as absorption with either soda lime or sodium hydroxide can be used. The preferred procedure for carbon dioxide removal is by contacting the gas stream with sodium hydroxide deposited on particles of vermiculite to effect the following reaction:

$$2NaOH + CO_2 \longrightarrow Na_2CO_3 + H_2O$$

This reaction is exothermic and will occur quantitatively at ambient temperature. The temperature rise is only a few degrees Celsius since there is only a small amount of carbon dioxide present. The reaction can be operated in the same range of temperature and pressure conditions as the oxidation of the carbon monoxide to carbon dioxide.

Since the reaction is not easily reversible, the sodium hydroxide-coated particles must be replaced after their capacity to remove carbon dioxide is exceeded. However, continuous operation of the process can be maintained by switching to an alternate bed while the spent bed is being discharged and refilled. The water that is generated in the reaction is bound to the sodium carbonate and unreacted sodium hydroxide as hydrates. However, at elevated temperatures these hydrates are decomposed. Therefore, the gas-stream from the reaction should be dried by passage through a bed of molecular sieves if minimum moisture content is desired.

Mixtures of $MnO_2$ and CuO in particulate form are commercially available, and they can be directly used in the present invention without further processing. However, heating with a purge gas, such as nitrogen, prior to using is useful to remove any traces of $H_2O$ that might be present. Particle size in the mixture is not especially critical, and can vary over a wide range. A mixture having a particle size range of 8—24 mesh (U.S. Sieve size) has worked well, but larger or smaller particle sizes can be used. Sodium hydroxide on vermiculite particles is also commercially available. Here again, particle size may vary. Material having a particle size range of 20—30 mesh (U.S. Sieve size) has proved effective in removing $CO_2$ from the perfluoroalkane as has material in the particle size range of 8—20 mesh (U.S. Sieve size). The larger size material is preferred since the finer material has more of a tendency to harden together upon prolonged $CO_2$ exposure, and this can eventually lead to plugging.

## Example

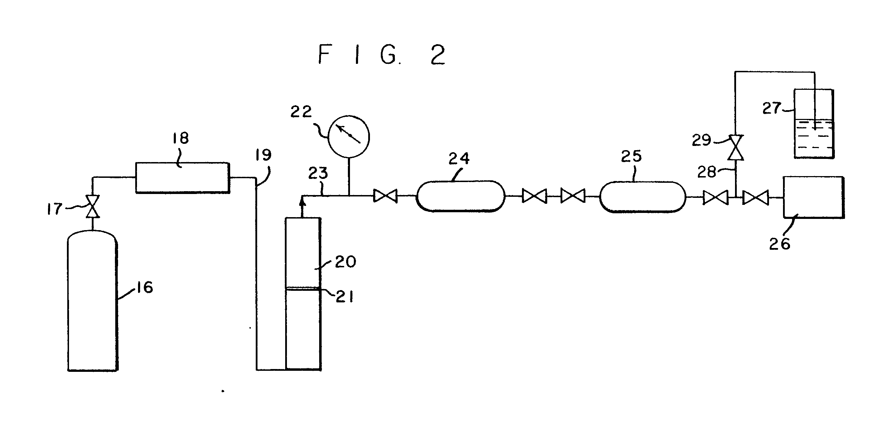

Referring to FIG. 2, tetrafluoromethane gas containing carbon monoxide and air was fed from gas cylinder *16* equipped with a valve *17* and flow meter *18* via line *19* to column *20* which was a 20 × 320 mm glass tube equipped with a vacuum jacket so that adiabatic measurements could be made. The bottom of the tube to its midpoint was filled with a mixture of manganese dioxide and cupric oxide granules having a 8—12 mesh (U.S. Sieve size) particles size to catalyze oxidation of carbon monoxide to carbon dioxide. A plug of glass wool *21* approximately 15 mm thick was situated on top of the bed of $MnO_2/CuO$ particles, and the remainder of the tube was filled with granules consisting of vermiculite particles coated with sodium hydroxide which served to absorb the $CO_2$ that was produced. The granules were 20—30 mesh (U.S. Sieve size) and were 92% by weight sodium hydroxide and 8% by weight vermiculite. Gas flow through the column *20* was upward. A pressure gauge *22* was located in exit line *23* of column *20* which was followed by a train of gas collecting and measuring devices which consisted of gas collecting bulbs *24* and *25*

with stopcocks at each end and moisture meter *26*. The gas bulbs were connected into the train by rubber tubing making it convenient to remove them for gas chromatograph analysis of their contents. Gas bulb *24* was employed for collecting samples for CO analysis while bulb *25* was employed for samples undergoing $CO_2$ analysis. At regular intervals, the exit stopcock on the first gas bulb was closed, and the pressure in the system was allowed to rise to 0.8—10.3 bar before closing the stopcock at the entrance end of the bulb thus providing gas samples under about two atmospheres of pressure. This was necessary since the analytical method for CO required pressure in the sample. The method used for CO analysis was sensitive to levels above 30 ppm. The $CO_2$ analysis was sensitive down to 3 ppm. A gas wash bottle *27* was connected into the train between gas bulb *25* and the moisture meter *26* by a shunt line *28* and valve *29*. This made it possible to sample the gas for measurement of acidity which would be indicative of tetrafluoromethane decomposition.

Six cylinders of tetrafluoromethane from plant production were used. To insure that carbon monoxide could be effectively removed from the tetrafluoromethane at the highest concentrations that might possibly be expected in crude product from plant production, additional carbon monoxide was added, and the CO level in each cylinder was determined. The CO levels in the cylinders were as follows: 1641 ppm; 1847 ppm; 1937 ppm; 2024 ppm; 2026 ppm and 2087 ppm. One cylinder (the one which contained 2026 ppm CO) was checked for oxygen content and was found to contain 1007 ppm $O_2$. The oxidation of carbon monoxide to carbon dioxide occurs according to the following equation:

$$2CO + O_2 \longrightarrow 2CO_2$$

Thus, it was concluded there was more than enough oxygen available to carry out this reaction. No oxygen was added to the tetrafluoromethane since air is inherently present in crude tetrafluoromethane in sufficient quantities to furnish the oxygen needed.

The bed of $MnO_2$/CuO particles was operated at ambient temperature, at 100°C, and at 200°C following heating with a dry purge of nitrogen gas until no evidence of $H_2O$ expulsion was detected. The oxidation of the CO was determined to be slightly exothermic.

The through-put rate was 0.56 kg of tetrafluoromethane per kg of the $MnO_2$/CuO mixture per hour. A total of 137 kg of tetrafluoromethane per kg of $MnO_2$/CuO mixture was treated, and the effluent gas from the bed was free of acids which was indicative that there was no decomposition of the tetrafluoromethane. No carbon monoxide was detected at any time during operation. The sodium hydroxide on the vermiculite eventually lost its effectiveness as it was consumed in the reaction with the carbon dioxide. However, no carbon dioxide was detected in the effluent gas until $CO_2$ constituted 13.5% by weight of the NaOH/vermiculite bed. Loss of $CO_2$ absorption activity could be qualitatively followed by the development of a white appearance moving through the bed.

**Claims**

1. A process for the removal of carbon monoxide from perfluoroalkanes comprising contacting a gaseous mixture of perfluoroalkane and carbon monoxide plus oxygen in a minor amount sufficient to oxidize carbon monoxide present in the perfluoroalkane to carbon dioxide with a bed of granules consisting of 67 to 86% by weight of manganese dioxide and 33 to 14% by weight of cupric oxide to convert the carbon monoxide to carbon dioxide and separating the carbon dioxide from the perfluoroalkane.

2. The process of Claim 1 in which the perfluoroalkane is tetrafluoromethane.

3. The process of Claim 1 in which the perfluoroalkane is hexafluoroethane.

4. The process of Claim 1 in which the perfluoroalkane is separated from the carbon dioxide by contacting the mixture of perfluoroalkane and carbon dioxide with a bed of particles consisting of sodium hydroxide deposited on particles of vermiculite.

5. The process of Claim 1 in which the perfluoroalkane is tetrafluoromethane.

6. The process of Claim 1 in which the perfluoroalkane is hexafluoroethane.

7. A process for the removal of carbon monoxide from perfluoroalkanes comprising contacting a gaseous mixture of perfluoroalkane and carbon monoxide plus oxygen in a minor amount sufficient to oxidize carbon monoxide present in the perfluoroalkane to carbon dioxide with a bed of granules consisting of 67 to 86% by weight of manganese dioxide and 33 to 14% by weight of cupric oxide to convert the carbon monoxide, separating the carbon dioxide from the perfluoroalkane according to the process of Claim 4 and then drying the vaporous perfluoroalkane by contact with a bed of molecular sieves.

8. The process of Claim 7 in which the perfluoroalkane is tetrafluoromethane.

9. The process of Claim 7 in which the perfluoroalkane is hexafluoroethane.

**Patentansprüche**

1. Verfahren zur Entfernung von Kohlenmonoxid aus Perfluoralkanen, umfassend die Inberührungbringung einer gasförmigen Mischung aus Perfluoralkan und Kohlenmonoxid zuzüglich Sauerstoff in einer geringen Menge, die ausreicht, um das in dem Perflouralkan anwesende Kohlenmonoxid zu Kohlendioxid zu oxidieren, mit einem Bett aus Granulat, bestehend aus 67 bis 86 Gew.-% Mangandioxid und 33 bis 14 Gew.-% Kupfer(II)-oxid, um das Kohlenmonoxid in Kohlendioxid zu überführen, und die Abtrennung des Kohlendioxids vom Perfluoralkan.

2. Verfahren nach Anspruch 1, bei welchem das Perfluoralkan Tetrafluormethan ist.

3. Verfahren nach Anspruch 1, bei welchem das Perfluoralkan Hexafluorethan ist.

4. Verfahren nach Anspruch 1, bei welchem das Perfluoralkan von dem Kohlendioxid abgetrennt wird, indem die Mischung aus Perfluoralkan und Kohlendioxid mit einem Bett von Teilchen in Berührung gebracht wird, die aus Vermiculitteilchen mit darauf abgelagertem Natriumhydroxid bestehen.

5. Verfahren nach Anspruch 4, bei dem das Perfluoralkan Tetrafluormethan ist.

6. Verfahren nach Anspruch 4, bei dem das Perfluoralkan Hexafluorethan ist.

7. Verfahren zur Entfernung von Kohlenmonoxid aus Perfluoralkanen, umfassend die Inberührungbringung einer gasförmigen Mischung aus Perfluoralkan und Kohlenmonoxid zuzüglich Sauerstoff in einer geringen Menge, die ausreicht, um das in dem Perfluoralkan anwesende Kohlenmonoxid zu Kohlendioxid zu oxidieren, mit einem Bett aus Granulat, bestehend aus 67 bis 86 Gew.-% Mangandioxid und 33 bis 14 Gew.-% Kupfer-(II)-oxid, um das Kohlenmonoxid in Kohlendioxid zu überführen, die Abtrennung des Kohlendioxids vom Perfluoralkan gemäß dem Verfahren nach Anspruch 4, und das anschließende Trocknen des gasförmigen Perfluoralkans durch Kontakt mit einem Bett von Molekularsieben.

8. Verfahren nach Anspruch 7, bei dem das Perfluoralkan Tetrafluormethan ist.

9. Verfahren nach Anspruch 7, bei dem das Perfluoralkan Hexafluorethan ist.

**Revendications**

1. Un procédé pour éliminer le monoxyde de carbone de perfluoroalcanes consistant à mettre un mélange gazeux der perfluoroalcane et de monoxyde de carbone contenant de l'oxygène en une quantité mineure suffisante pour oxyder en anhydride carbonique le monoxyde de carbone présent dans le perfluoroalcane, en contact avec un lit de granules composés de 67 à 86% en poids de bioxyde de manganèse et 33 à 14% en poids d'oxyde cuivrique pour transformer le monoxyde de carbone en anhydrixde carbonique, et à séparer l'anhydride carbonique du perfluoroalcane.

2. Le procédé de la revendication 1, dans lequel le perfluoroalcane est le tétrafluorométhane.

3. Le procédé de la revendication 1, dans lequel le perfluoroalcane est l'hexafluoroéthane.

4. Le procédé de la revendication 1, dans lequel le perfluoroalcane est séparé de l'anhydride carbonique par mise en contact du mélange de perfluoroalcane et d'anhydride carbonique avec un lit de particules composées d'hydroxyde de sodium déposé sur des particules de vermiculite.

5. Le procédé de la revendication 1, dans lequel le perfluoroalcane est le tétrafluorométhane.

6. Le procédé de la revendication 4, dans lequel le perfluoroalcane est l'hexafluoroéthane.

7. Un procédé pour éliminer le monoxyde de carbone de perfluoroalcanes consistant à mettre un mélange gazeux de perfluoroalcane et de monoxyde de carbone contenant de l'oxygène en une quantité mineure suffisante pour oxyder en anhydride carbonique le monoxyde de carbone présent dans le perfluoroalcane, en contact avec un lit de granules composés de 67 à 86% en poids de bioxyde de manganèse et 33 à 14% en poids d'oxyde cuivrique pour transformer le monoxyde de carbone, à séparer l'anhydride carbonique du perfluoroalcane conformément au procédé de la revendication 4, puis à sécher le perfluoroalcane gazeux par mise en contact avec un lit de tamis moléculaires.

8. Le procédé de la revendication 7, dans lequel le perfluoroalcane est le tétrafluorométhane.

9. Le procédé de la revendication 7, dans lequel le perfluoroalcane est l'hexafluoroéthane.

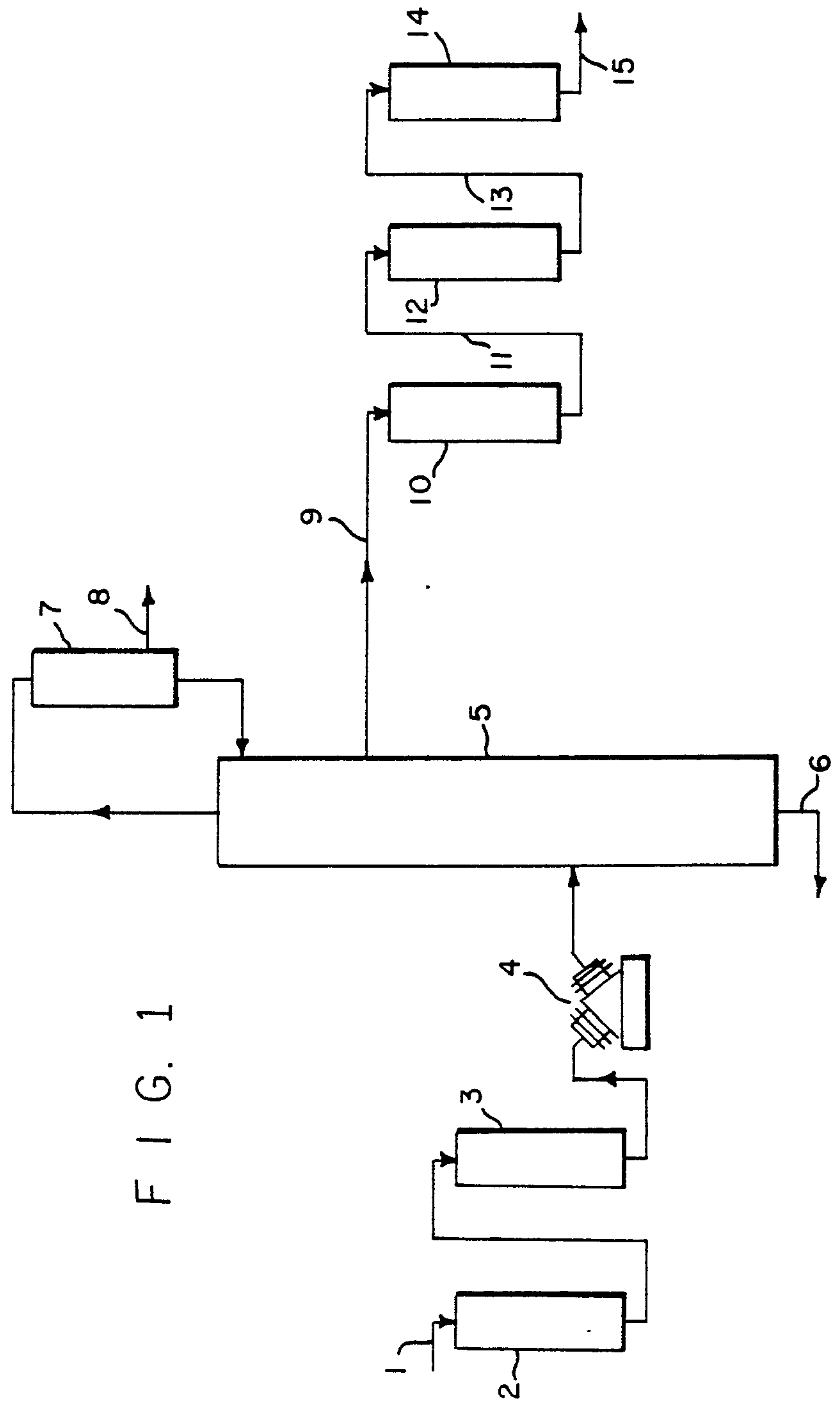


F I G. 1

FIG. 2